# FASCICULE DE BREVET EUROPEEN

(11) **EP 1 039 913 B1**
(45) Date de publication et mention de la délivrance du brevet: **15.09.2004**
(21) Numéro de dépôt: 98962514.0
(22) Date de dépôt: 18.12.1998
(51) Int. Cl.: A61K 31/57, A61K 9/20

(54) **COMPRIME DE PROGESTERONE ET SON PROCEDE DE PREPARATION**
PROGESTERONTABLETTE UND VERFAHREN ZU DEREN HERSTELLUNG
PROGESTERONE TABLET AND PREPARATION METHOD

(30) Priorité: 19.12.1997 FR 9716168
(43) Date de publication de la demande: 04.10.2000
(73) Titulaire: Laboratoires BESINS ISCOVESCO Société anonyme dite :, 75003 Paris (FR)
(72) Inventeur: AGNUS, Benoît, F-94360 Bry sur Marne (FR); BESINS, Antoine, F-75017 Paris (FR)
(74) Mandataire: Nargolwalla, Cyra
(86) Numéro de dépôt international: PCT/FR1998/002790
(87) Numéro de publication internationale: WO 1999/032126

(56) Documents cités:
- EP-A- 0 330 284
- WO-A-95/05807
- FR-A- 2 383 664
- US-A- 4 911 921

## Description

La présente invention a pour objet un comprimé de progestérone, ainsi qu'un procédé pour sa préparation.

La progestérone naturelle est une hormone qui est synthétisée, chez la femme, essentiellement par l'ovaire et, à un moindre degré, par les glandes surrénales et le placenta au cours de la deuxième partie de la grossesse. Une synthèse non endocrine de progestérone, notamment au niveau des neurones, est également possible.

L'insuffisance de la sécrétion de la progestérone chez une femme peut conduire à des troubles divers, en particulier :
- syndromes prémenstruels,
- irrégularités menstruelles par disovulation ou anovulation,
- mastopathies bénignes,
- préménopause,
- ménopause.

Pour cette dernière indication, la progestérone est administrée en complément d'un traitement oestrogénique.

L'administration par voie orale de la progestérone est sévèrement compromise en raison de sa métabolisation importante par le foie.

Or, le mode d'administration par voie orale présente des avantages évidents par rapport à d'autres méthodes d'administration. En effet, elle est d'une part plus pratique que l'administration par voie vaginale, et d'autre part, elle permet une prise indépendante qui est impossible lorsqu'il s'agit de l'administration par voie parentérale.

La Société Demanderesse a déjà proposé une solution à ce problème de dégradation de la progestérone, dans la demande de brevet FR 76 36007. En effet, elle a développé une formulation de capsules contenant de la progestérone en suspension huileuse, ce qui assurait une bonne protection du principe actif contre sa dégradation par le foie.

Le procédé de préparation de telles capsules se révèle cependant d'une mise en oeuvre complexe et coûteuse, nécessitant en plus un savoir faire considérable. On a donc essayé de développer des formulations alternatives efficaces, mais également économiquement viables.

C'est ainsi que la Société PHARMAGYN décrit dans le brevet européen n° 0 335 970 des comprimés contenant de la progestérone micronisée en association avec une cire, toutes deux sous forme pulvérulente. Il est indiqué dans ce brevet que le fait d'inclure des quantités importantes d'une cire dans les comprimés parvient à limiter la dégradation de la progestérone par le foie, augmentant de ce fait sa biodisponibilité.

De même, la Société NOVO NORDISK, dans la demande de brevet internationale WO95/05807, décrit des comprimés contenant de la progestérone, un polyéthylène glycol, ainsi qu'un excipient choisi dans le groupe comprenant les amidons, les composés d'amidon, les amidons modifiés, les celluloses, les celluloses modifiées, les pectines et la tragacanthe. Il est indiqué dans ce document que la présence du polyéthylène glycol et de l'excipient dans les comprimés a pour conséquence un effet favorable sur la biodisponibilité de la progestérone administrée par voie orale.

Il convient de souligner que les comprimés décrits dans ces deux documents contiennent de forts pourcentages en excipients. En effet, les comprimés selon les exemples du brevet EP 0 335 970 contiennent 68% d'excipients tandis que ceux de la demande de brevet WO 95/05807 contiennent d'environ 49% à 62% d'excipients. Le document EP 0 330 284 A2 décrit des comprimés à haute teneur en progestérone contenant de la cellulose microcristalline (AVICEL) comme agent liant.

Les excipients dans les comprimés, on le sait, jouent des rôles divers. Ils servent à augmenter la stabilité des principes actifs, à permettre l'obtention d'un profil de libération particulier selon leur nature, mais ils servent surtout à faciliter la compression des différents ingrédients afin d'obtenir un comprimé possédant des bonnes caractéristiques de dureté, de désagrégation, et de dissolution.

Or, après de longs et nombreux travaux et recherches, la Société Demanderesse est parvenue à mettre au point un nouveau comprimé de progestérone naturelle ne contenant que de faibles quantités d'excipients.

Et de façon tout à fait surprenante et inattendue, les comprimés mis au point par la Société Demanderesse, bien que contenant des quantités d'excipients significativement inférieures à celles des comprimés de l'art antérieur, présentent néanmoins d'excellentes caractéristiques de stabilité, de dureté, de désagrégation et de dissolution.

La présente invention concerne donc un comprimé de progestérone, ce comprimé étant caractérisé par le fait que sa teneur en excipients est d'au plus 45%, de préférence d'au plus 40%, et plus préférentiellement encore d'au plus 38 %, les pourcentages étant exprimés en poids par rapport à la matière sèche totale du comprimé, et par le fait qu'il contient au moins un polyvinylpyrrolidone comme agent liant.

Le comprimé selon l'invention contient, outre la progestérone, des agents diluants, des agents désintégrants, des agents lubrifiants et des agents liants, dont au moins un polyvinylpyrrolidone.

Comme exemples d'agents diluants on peut citer les amidons, les polyols et les celluloses. De préférence, le comprimé selon l'invention contient de l'amidon de maïs prégélatinisé, du mannitol et de la cellulose microcristalline.

L'amidon de maïs prégélatinisé est un amidon qui a été physiquement modifié afin de permettre un écoulement fluide et être directement compressible. Il présente donc de multiples avantages en ce qui concerne la fabrication de comprimés, dont notamment l'amélioration de la dissolution et l'auto-lubrification.

La cellulose microcristalline présente de bonnes qualités d'écoulement libre. Elle présente en outre de bonnes propriétés comme agent liant et désintégrant, étant ainsi un excipient idéal pour la fabrication des comprimés par compression directe.

Comme exemples d'agents désintégrants on peut citer les carboxyméthylcelluloses, l'acide alginique ainsi que son sel de sodium, et les amidons. De préférence, le comprimé conforme à l'invention contient de la carboxyméthylcellulose sodique réticulée. Ce produit a été retenu grâce à ses qualités d'agent désintégrant, ainsi que pour son fort pouvoir absorbant.

L'agent lubrifiant préféré dans le cadre de la présente invention est le stéarate de magnésium.

Parmi les agents liants préférés dans le cadre de la présente invention on peut citer les polyvinylpyrollidones, en particulier le produit vendu sous la marque Polyvidone K30. Ce produit permet une libération quasi-immédiate du principe actif, étant en plus d'une mise en oeuvre très aisée.

Un des avantages du comprimé objet de l'invention est qu'il présente un temps de désagrégation inférieur à 15 minutes, de préférence inférieur à 10 minutes, et plus préférentiellement encore inférieur à 5 minutes.

Un autre avantage du comprimé selon la présente invention est qu'il présente un profil de dissolution tel que la teneur en progestérone libérée est d'au moins de 75 % en 15 minutes, de préférence en 10 minutes, et plus préférentiellement encore en 5 minutes.

Le comprimé selon l'invention présente une dureté comprise entre 20 et 120 N, de préférence comprise entre 30 et 110 N, et plus préférentiellement encore comprise entre 40 et 100 N.

L'invention concerne également un procédé pour la préparation de comprimés de progestérone. Ce procédé est caractérisé par le fait que :
- on prépare un premier mélange de progestérone et de diluant,
- on prépare une suspension de mouillage, contenant le polyvinylpyrrolidone,
- on procède au mouillage du premier mélange par la suspension de mouillage,
- on procède à une granulation du produit du mouillage, afin d'obtenir un produit granulaire,
- on ajoute des agents désintégrants et diluants au produit granulaire afin d'obtenir un deuxième mélange,
- on ajoute un lubrifiant au deuxième mélange afin d'obtenir un troisième mélange,
- on effectue une compression de ce troisième mélange afin d'obtenir des comprimés.

Selon un mode préférentiel de réalisation de l'invention, la granulation est suivie par un démottage, un séchage, puis un calibrage.

L'invention sera mieux comprise à l'aide des exemples non limitatifs décrits ci-dessous.

### EXEMPLE 1 : COMPRIMES DE PROGESTERONE

Les compositions de comprimés de progestérone selon l'invention contenant respectivement 200 et 100 mg de principe actif sont données au Tableau 1 ci-dessous.

**TABLEAU I**

| NOM DU COMPOSANT | FONCTION | QUANTITE mg/comprimé | |
|---|---|---|---|
| Progestérone | Principe actif (progestatif) | 200 | 100 |
| Polyvidone K30 | Liant | 9,60 | 4,80 |
| Amidon de maïs prégélatinisé | Diluant | 92,80 | 46,40 |
| Carboxyméthyl cellulose sodique réticulée | Désintégrant | 16,00 | 8,00 |
| Stéarate de magnésium | Lubrifiant | 1,60 | 0,80 |

### EXEMPLE 2 : PREPARATION DE COMPRIMES DE PROGESTERONE

Un lot de comprimés contenant 100 mg de progestérone par comprimé a été préparé comme décrit ci-dessous.

### a/ Préparation du mélange de progestérone

On mélange 30 kg de progestérone micronisée (obtenues auprès de la société DIOSYNTH, à Oss, Pays Bas) et 9,6 kg d'amidon de maïs prégélatinisé dans la cuve d'un mélangeur granulateur planétaire du type BONNET équipé d'un axe d'agitation du type Lyre, pendant 10 minutes, afin d'obtenir un mélange homogène.

### b/ Préparation de la suspension de mouillage

On introduit 5,76 1 de l'eau purifiée dans un récipient en acier inoxydable. On agite à l'aide d'un mélangeur-malaxeur TURBOTEST RAYNERI équipé d'un axe d'agitation de type défloculeuse.

On y verse progressivement 1,44 kg de polyvidone K30 (commercialisé sous la marque KOLLIDON par la Société BASF), et puis on homogénéise jusqu'à la dissolution totale du polyvidone.

### c/ Mouillage

On verse la solution de mouillage en filet sur le mélange de poudre contenu dans le mélangeur BONNET, l'axe de type Lyre étant programmé sur la vitesse 1, pendant 30 minutes.

### d/ Granulation

On homogénéise le mélange pendant 15 minutes après la fin du mouillage afin d'obtenir une poudre granulaire.

### e/ Séchage

Le séchage est pratiqué dans une étuve à 40°C pendant 6 heures.

### f/ Calibrage

Le calibrage de la poudre granulaire est réalisé à l'aide d'un granulateur oscillant FREWITT équipé d'une grille d'inox de diamètre de maille de 1000µm.

### g/ Ajout des agents désintégrants et diluants au produit granulaire

A 39,6 kg du produit granulaire on ajoute 4,168 kg d'amidon prégélatinisé (AMIDON 1500), et 2,316 kg de carboxyméthylcellulose réticulée dans la cuve d'un mélangeur ROUE RÖHN et on mélange pendant deux fois cinq minutes.

Au mélange ainsi obtenu on ajoute 0,232 kg de stéarate de magnésium et on mélange pendant trois minutes afin d'obtenir le mélange final.

### h/ compression du mélange final

La compression du mélange final est effectuée sur une machine à comprimer rotative de type FROGERAIS MR 200, équipée de poinçons. Le réglage de la machine est effectué de manière à obtenir pour des comprimés de masse unitaire de 160 mg un temps de désagrégation inférieur à environ 5 minutes et une dureté d'environ 40 N.

Les comprimés obtenus sont conditionnés en plaquettes alvéolées, thermoformées, constituées d'une feuille de PVC d'une épaisseur de 250 µm, scellée à une feuille d'aluminium d'une épaisseur de 20 µm.

Une étude de stabilité a été réalisée sur les comprimés ainsi préparés.

Ils ont été stockés pendant 30 jours à 45°C dans des conditionnements en verre teinté étanches.

Aucune modification des paramètres galéniques et analytiques n'a été observée à la fin de cette période, démontrant ainsi la bonne stabilité des comprimés selon l'invention.

### EXEMPLE 3 : TEMPS DE DESAGREGATION DES COMPRIMES SELON L'INVENTION

Un comprimé préparé selon la méthode décrite dans l'exemple 2 a été placé dans un bécher contenant 600 ml d'eau distillée, portés à une température de 37°C. Le temps de désagrégation correspondant à une perte de cohésion totale était de 3 minutes.

### EXEMPLE 4 : TEMPS DE DISSOLUTION DES COMPRIMES SELON L' INVENTION

On a pris 6 cuves de dissolution de capacité 1000 ml. Dans chacune des cuves on a placé un comprimé dans un milieu de dissolution contenant 1000 ml d'une solution aqueuse contenant 1 % (m/v) de sodium laurylsulfate. On a ensuite agité la solution dans chaque cuve à l'aide d'un appareil de dissolution à pales tournantes PHARMATEST type PTWS III. Les pales ont été immergées dans le milieu de dissolution à une distance de 25 mm ± 2 mm entre la pale et le fond de la cuve. Les pales ont été mises sous une agitation de 75 tours par minute.

1 ml du milieu de dissolution a été prélevé toutes les 5 minutes dans chacune des cuves.

Chaque échantillon a été dosé par HPLC (= 240 ηm) après injection de 20 µl de solution à analyser.

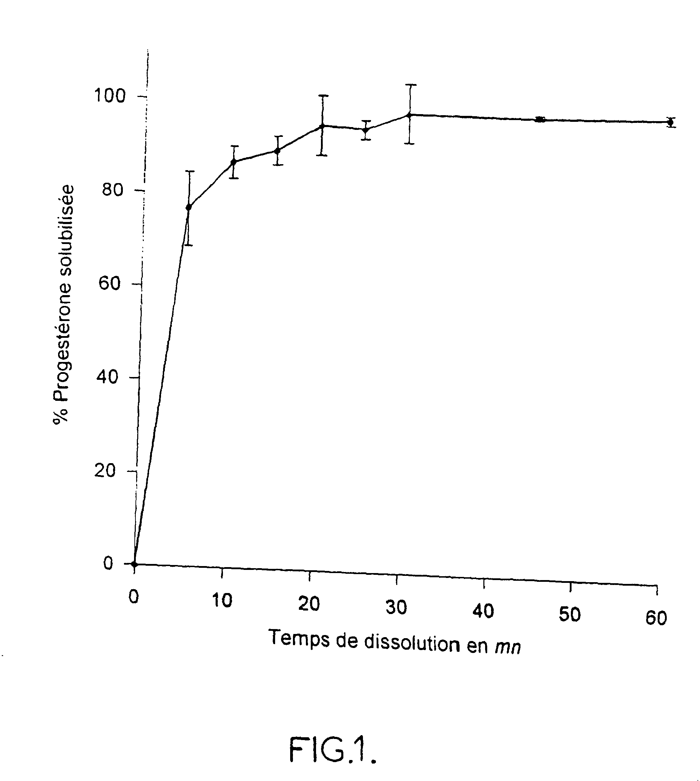

La courbe donnée dans la Figure 1 démontre le profil de dissolution obtenu. Au moins 75 % de la progestérone est libérée à 15 minutes de dissolution.

### EXEMPLE 5 : BIODISPONIBILITE DE LA PROGESTERONE CONTENUE DANS LES COMPRIMES SELON L'INVENTION

Une étude pharmacocinétique a été réalisée sur cinq patientes afin de démontrer la libération de la progestérone contenue dans des comprimés de 100 mg ayant la composition décrite dans le tableau I ci-dessus.

Le Tableau II ci-dessous donne les concentrations de progestérone libérée dans le sang pendant des intervalles de temps indiquées, jusqu'à 24 heures après l'administration du comprimé.

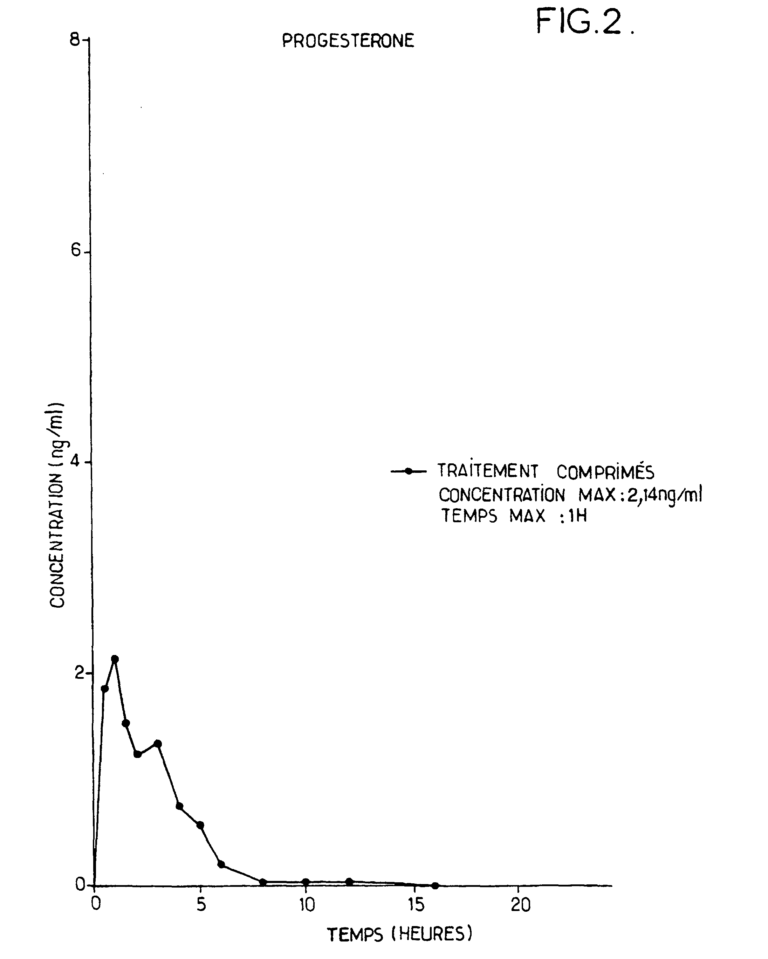

La Figure 2 montre une moyenne de la concentration de progestérone libérée dans le sang des cinq patientes.

Ces résultats démontrent la bonne biodisponibilité de la progestérone contenue dans les comprimés selon l'invention.

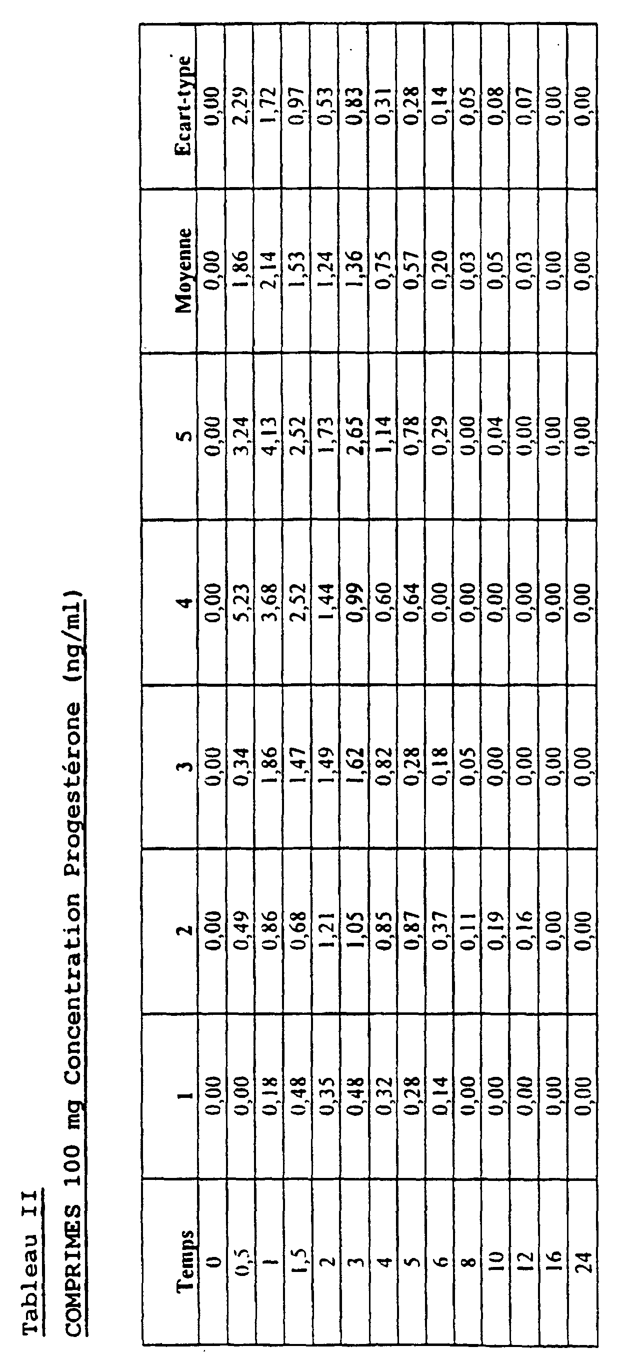

## Revendications

1. Comprimé de progestérone, **caractérisé par le fait que** sa teneur en excipients est d'au plus 45%, de préférence d'au plus 40%, et plus préférentiellement encore d'au plus 38 %, les pourcentages étant exprimés en poids par rapport a la matière sèche totale du comprimé, et **par le fait qu'**il contient au moins un polyvinylpyrrolidone comme agent liant.

2. Comprimé de progestérone selon la revendication 1, **caractérisé par le fait qu'**il présente un temps de désagrégation inférieur à 15 minutes, de préférence inférieur à 10 minutes, et plus préférentiellement encore inférieur à 5 minutes.

3. Comprimé de progestérone selon l'une ou l'autre des revendications 1 et 2, **caractérisé par le fait qu'**il présente un profil de dissolution tel que la teneur en progestérone libérée est d'au moins 75 % en 15 minutes, de préférence en 10 minutes, et plus préférentiellement encore en 5 minutes.

4. Comprimé de progestérone salon l'une quelconque des revendications 1 à 3, **caractérisé par le fait qu'**il présente une dureté comprise entre 20 et 120 N, de préférence comprise entre 30 et 110 N, et plus préférentiellement encore comprise entre 40 et 100 N.

5. Comprimé de progestérone selon l'une quelconque des revendications 1 à 4, **caractérisé par le fait qu'**il contient, outre la progestérone et au moins un polyvinylpyrrolidone, des agents diluants, des agents désintégrants, des agents lubrifiants et des agents liants.

6. Procédé de préparation d'un comprimé selon l'une quelconque des revendications 1 à 5, **caractérisé par le fait que** :
- on prépare un premier mélange de progestérone et de diluant,
- on prépare une suspension de mouillage contenant le polyvinylpyrrolidone,
- on procède au mouillage du premier mélange par la suspension de mouillage,
- on procède à une. granulation du produit du mouillage, afin d'obtenir un produit granulaire,
- on ajoute des agents désintégrants et diluants au produit granulaire afin d'obtenir un deuxième mélange,
- on ajoute un lubrifiant au deuxième mélange afin d'obtenir un troisième mélange,
- on effectue une compression de ce troisième mélange afin d'obtenir des comprimés.

## Patentansprüche

1. Progesterontablette, **dadurch gekennzeichnet, dass** ihr Gehalt an Arzneimittelträgern höchstens 45%, vorzugsweise höchstens 40% und noch stärker bevorzugt höchstens 38% beträgt, wobei die Prozentsätze in Gewichtsprozenten im Verhältnis zum gesamten Trockenmaterial der Tablette ausgedrückt sind, und **dadurch gekennzeichnet, dass** sie mindestens ein Polyvinylpyrrolidon als Bindemittel enthält.

2. Progesterontablette nach Anspruch 1, **dadurch gekennzeichnet, dass** sie eine Dessaggregationszeit von weniger als 15 Minuten, vorzugsweise weniger als 10 Minuten und noch weiter bevorzugt von weniger als 5 Minuten aufweist.

3. Progesterontablette nach einem der Ansprüche 1 oder 2, **dadurch gekennzeichnet, dass** sie ein solches Auslösungsprofil aufweist, dass der Gehalt an freigesetzter Progesteron mindestens 75% in 15 Minuten beträgt, vorzugsweise in **10** Minuten und noch weiter bevorzugt in 5 Minuten.

4. Progesterontablette nach einem der Ansprüche 1 bis 3, **dadurch gekennzeichnet, dass** sie eine Härte von 20 bis 120 N, vorzugsweise von 30 bis 110 N und noch weiter bevorzugt von 40 bis 100 N aufweist.

5. Progesterontablette nach einem der Ansprüche 1 bis 4, **dadurch gekennzeichnet, dass** sie - außer Progesteron und mindestens einem Polyvinylpyrrolidon-Verdünnungsmittel, Auflösungsmittel, Gleitmittel und Bindemittel enthält.

6. Verfahren zur Herstellung einer Tablette nach einem der Ansprüche 1 bis 5, **dadurch gekennzeichnet, dass**:
- eine erste Mischung aus Progesteron und Verdünnungsmittel hergestellt wird,
- eine Benetzungssuspension, die Polyvinylpyrrolidon enthalt, hergestellt wird,
- eine Benetzung der ersten Mischung durch die Benetzungssuspension durchgeführt wird,
- eine Granulierung des Produkts der Benetzung durchgeführt wird, bis ein granuläres Produkt erhalten wird,
- Auflösungsmittel und Verdünnungsmittel zum granulären Produkt hinzugefügt. werden, bis eine zweite Mischung erhalten wird,
- ein Gleitmittel zur zweiten Mischung hinzugefügt wird, bis eine dritte Mischung erhalten wird,
- eine Kompression dieser dritten Mischung vorgenommen wird, bis Tabletten erhalten werden.

## Claims

1. Progesterone tablet, **characterised in that** its excipients content is at most 45%, preferably at most 40%, and yet more preferable at most 38%, the percentages being expressed in weight relative to the total dry matter of the tablet, and **in that** it contains at least one polyvinylpyrrolidone as a binding agent.

2. Progesterone tablet according to claim 1, **characterised in that** it has a disintegration time of less than 15 minutes, preferably less than 10 minutes, and yet more preferably less than 5 minutes.

3. Progesterone tablet according to one or the other of claims and **2, characterised in that** it has a dissolution profile such that the level of progesterone released is at least 75% in 15 minutes, preferably in 10 minutes, and yet more preferably in 5 minutes.

4. Progesterone tablet according to any one of claims 1 to 3, **characterised in that** it has a hardness between 20 and 120 N, preferably between 30 and 110 N, yet more preferably between 40 and 100 N.

5. Progesterone tablet according to any of claims 1 to 4, **characterised in that**, besides progesterone and at least one polyvinylpyrrolidone, the tablet contains diluents, disintegrating agents, lubricants and binding agents.

6. Manufacturing process of a tablet according to any of claims 1 to 5, **characterised in that**:
- a first mixture of progesterone and diluent is prepared,
- a wetting suspension containing the polyvinylpyrrolidone is prepared,
- the first mixture is then wetted by the wetting suspension,
- the product of the wetting is then granulated in order to obtain a granular product,
- disintegrating agents and diluents are added to the granular product in order to obtain a second mixture,
- a lubricant is added to the second mixture in order to obtain a third mixture,
- this third mixture is compressed in order to obtain tablets.
